# EUROPÄISCHE PATENTSCHRIFT

(11) **EP 2 380 535 B1**
(45) Veröffentlichungstag und Bekanntmachung des Hinweises auf die Patenterteilung: **08.04.2015**
(21) Anmeldenummer: 10004224.1
(22) Anmeldetag: 21.04.2010
(51) Int. Cl.: A61B 18/00, A61F 9/008, A61B 18/20, A61F 9/007, A61N 5/06

(54) **Vorrichtung für die Vernetzung von okulärem Gewebe mit elektromagnetischer Strahlung**
Device for integrating ocular tissue with electromagnetic radiation
Dispositif de réticulation d'un tissu oculaire à l'aide d'un rayonnement électromagnétique

(43) Veröffentlichungstag der Anmeldung: 26.10.2011
(73) Patentinhaber: IROC Innocross AG, 6300 Zug (CH)
(72) Erfinder: Mrochen, Michael, 8002 Zürich (CH); Seiler, Theo, 8002 Zürich (CH)
(74) Vertreter: von Hellfeld, Axel

(56) Entgegenhaltungen:
- EP-A1- 1 561 440
- EP-A1- 1 790 383
- WO-A2-2008/008914
- DE-A1-102006 030 219
- DE-U1-202009 015 776

## Beschreibung

Die Erfindung betrifft eine Vorrichtung für die Vernetzung von okulärem Gewebe mit elektromagnetischer Strahlung, insbesondere UV-Strahlung, wobei durch die Vernetzung mittels eines in das okuläre Gewebe eingebrachten Fotosensibilisators die biomechanischen Eigenschaften des okulären Gewebes geändert werden, mit Mitteln zum Einstrahlen der elektromagnetischen Strahlung in das Gewebe, wobei die eingestrahlte elektromagnetische Strahlung den Fotosensibilisator für eine Vernetzung aktiviert.

Der menschliche Augapfel wird durch die äußere Augenhaut begrenzt. Durch den Augeninnendruck wird die kollagenhaltige äußere Außenhaut gespannt und verleiht dem gesunden Augapfel eine annähernd sphärische Form. Im hinteren Augapfelbereich besteht die äußere Augenhaut aus der weißen Lederhaut (Sklera). Im vorderen Bereich befindet sich die für sichtbares Licht durchlässige Hornhaut (Kornea).

Verformungen der äußeren Augenhaut können Ursache einer Fehlsichtigkeit sein. Beispielsweise kann eine Form der Kurzsichtigkeit, die Achsenmyopie, Folge eines skleraren Längenwachstums des Augapfels sein. Eine als Ellipsoid geformte Oberfläche der Kornea kann zu einer Form des Astigmatismus führen, welcher auch als Hornhautverkrümmung bezeichnet wird. Eine weitere Erkrankung der Hornhaut wird als Keratokonus bezeichnet. Beim Keratokonus kommt es infolge einer krankhaften Erweichung der Hornhaut zu einer fortschreitenden Ausdünnung und kegelförmigen Verformung der Hornhaut des Auges. Mit der zunehmenden Auswölbung wird die Hornhaut unterhalb des Zentrums dünner. Sie kann durchbrechen und vernarben. Das setzt die Sehschärfe dauerhaft herab. Die Ursachen des Keratokonus sind heutzutage noch weitgehend unbekannt. Er tritt familiär gehäuft auf, was unter anderem auf eine genetische Disposition schließen lässt. Einen weiteren Risikofaktor für die Entstehung eines Keratokonus stellen Atopien, wie allergische Erkrankungen, dar.

Die konventionelle Therapie eines fortgeschrittenen Keratokonus sieht vor, die erkrankte Kornea zu entfernen und durch ein allogenes Transplantat zu ersetzen. Eine solche Operation ist jedoch eine Organverpflanzung mit den damit verbundenen Risiken und Komplikationen. Ein angemessenes Sehvermögen wird häufig erst ca. zwei Jahre nach der Operation erreicht. Zudem betrifft die Hornhautverpflanzung beim Keratokonus meist junge Menschen, weshalb das Transplantat über Jahrzehnte hinweg einwandfrei funktionieren muss.

Eine demgegenüber verbesserte Therapie des Keratokonus stabilisiert die Kornea durch Vernetzung. Die Behandlung erlaubt eine photochemische, nichtgewebeabtragende Stabilisierung oder Veränderung der biomechanischen und biochemischen Eigenschaften der Kornea. Das Behandlungsprinzip ist auch auf andere betroffene Regionen des Auges anwendbar. Eine Photosensibilisatorlösung wird in das zu verändernde Augengewebe eingebracht und einer Primärstrahlung ausgesetzt. Als Primärstrahlung wird elektromagnetische Strahlung im Wellenlängebereich von etwa 300 nm bis 800 nm (UV-A-Strahlung oder sichtbares Licht) eingesetzt.

Entsprechende Vorrichtungen zur Behandlung der äußeren Augenhaut sind aus den Druckschriften WO 2007/128581 A2 und WO 2008/000478 A1 bekannt.

Die EP 1 561 440 B1 beschreibt eine Vorrichtung, bei der mit einem relativ komplexen Aufbau eine homogene Verteilung der Strahlung im okulärem Gewebe erzeugt wird. Ein Formkörper wird dort auf die Hornhaut aufgesetzt, um diese in eine gewünschte Form zu bringen während mittels elektromagnetischer Strahlung und des Photosensibilisators das okuläre Gewebe hinsichtlich seiner Festigkeit geändert wird. Ein solcher Formkörper kann auch im Zusammenhang mit der vorliegenden Erfindung eingesetzt werden.

Eine Vorrichtung gemäß WO 2007/128581 A2 dient der Verfestigung der sich im hinteren Augenabschnitt befindenden Sklera. Die Primärstrahlung kann dabei durch das Innere des Auges oder durch von außen aufliegenden Kissen auf die Sklera einwirken. Durch einen Photomediator oder Photosensibilisator wird eine Vernetzung in der Sklera bewirkt. Dadurch wird einem Sklerawachstum entgegengewirkt und ein Fortschreiten der Achsenmyopie unterbunden.

Die EP 1 854 438 A1 beschreibt eine ophthalmalogische Vorrichtung zur Vorbeugung einer Myopie, bei der die Sklera mittels eines Photosensibilisators verfestigt wird.

Die Druckschriften WO 2008/000478 A1 und WO 2008/008914 A2 beschreiben Bestrahlungssysteme zur biomechanischen Stabilisierung der Kornea. Auch hier kann in Verbindung mit einem Photosensibilisator eine Vernetzung an der Kornea bewirkt werden. Das aus WO 2008/000478 A1 bekannte Bestrahlungssystem bietet die Möglichkeit spezifische Erkrankungen, wie den Keratokonus zu therapieren.

Die Änderung der Gestalt und/oder von mechanischen Eigenschaften von Augengewebe, insbesondere der Hornhaut und allgemein der Sklera mittels eines eingebrachten Photosensibilisators und elektromagnetischer Strahlung ist als solche im Stand der Technik, insbesondere wie oben genannt, gut bekannt. Hinsichtlich der chemischen Zusammensetzung des Photosensibilisators wird auf den Stand der Technik verwiesen, auch hinsichtlich der eingesetzten Art der elektromagnetischen Strahlung, insbesondere der geeigneten Wellenlängen in Verbindung mit bestimmten Photosensibilisatoren.

Einem routinemäßigen Einsatz der Vernetzungstherapie am Augengewebe stehen jedoch komplexe Abhängigkeiten entgegen. Die Beziehungen zwischen den eingesetzten Dosen und deren Wirkung im Augengewebe sind sehr vielfältig. Als Dosis in diesem Sinne kommen insbesondere in Betracht die elektromagnetische Strahlung hinsichtlich ihrer Intensität sowie ihrer Verteilung in Raum und Zeit; der eingesetzte Photosensibilisator hinsichtlich seiner chemischen Struktur, Konzentration, und Einwirkung in Raum und Zeit. Die Wirkungen von unterschiedlichen Dosen dieser Parameter auf und in dem Augengewebe eines Patienten sind sehr stark abhängig von Eigenschaften (Messdaten) bezüglich des Patienten. Dabei ist insbesondere zu berücksichtigen, dass die Wirkung der mit der Strahlung und dem Photosensibilisator durchgeführten Vernetzung auch unerwünscht sein kann bis hin zu einer Schädigung des Augengewebes oder der Funktion des Auges.

Der Erfindung liegt die Aufgabe zugrunde, eine Vorrichtung der eingangs genannten Art bereitzustellen, mit der die biomechanischen Eigenschaften von okulärem Gewebe gezielt und präzise geändert werden können.

Hierzu stellt die Erfindung eine Vorrichtung wie inden Ansprüchen definiert bereit.

Gemäß einer bevorzugten Ausgestaltung der Erfindung ist das genannte okuläre Gewebe die Kornea des Auges.

Eine weitere bevorzugte Ausgestaltung der Erfindung sieht vor, dass die genannte elektromagnetische Strahlung ganzflächig in die Kornea eingestrahlt wird, d. h. die Bestrahlung der Kornea erfolgt im Wesentlichen über deren gesamte ophthalmalogisch relevante Vorderfläche. Dies ermöglicht eine einfache Auf- und Einbringung des Photosensibilisators in das okuläre Gewebe und auch eine einfache Ausgestaltung der optischen Mittel für die Einstrahlung der elektromagnetischen Strahlung.

Bei Einsatz der bekannten Photosensibilisatoren handelt es sich bei der elektromagnetischen Strahlung regelmäßig um UV-Strahlung, so auch hier.

Je nach der medizinischen Indikation ermöglicht die Erfindung eine inhomogene Verteilung der elektromagnetischen Strahlung und dadurch auch eine gewünschte Verteilung der Vernetzung sowie der damit einhergehenden Änderungen in den biomechanischen Eigenschaften des okulären Gewebes. Damit lässt sich gezielt und präzise eine Änderung der Form des bestrahlten okulären Gewebes erreichen, da, in Abhängigkeit von den jeweiligen örtlichen biomechanischen Eigenschaften der Augeninnendruck eine unterschiedliche Wirkung bezüglich der Form des Gewebes hat. Stärker vernetzte Bereiche des Gewebes, d. h. stärker "verhärtete" Bereiche, zeigen im Vergleich zu weicheren Gewebebereichen eine geringere Auswölbung.

Die Erfindung ermöglicht auch eine Berücksichtigung und Kompensation der Winkel zwischen der Einstrahlungsrichtung der elektromagnetischen Strahlung und der Oberfläche des okulären Gewebes, also insbesondere der Kornea. Dabei berücksichtigt die Erfindung, dass die elektromagnetische Strahlung in Abhängigkeit vom Auftreffort auf die Oberfläche des Gewebes erstens ortsabhängig unterschiedlich reflektiert wird (in Abhängigkeit vom Einfallswinkel) und zweitens eine unterschiedliche Strahlungsstromdichte (*irradiance*) hat. Zum Beispiel wird die Strahlung in weiter von der optischen Achse des Systems entfernt liegenden äußeren Bereichen der Kornea (also Bereichen, die auch weiter von der Sehachse des Auges entfernt sind) aufgrund des flacheren Einfallswinkels stärker reflektiert als in zentralen Bereichen der Kornea, was zur Folge hat, dass die Strahlung in den äußeren, von der Achse entfernteren Bereichen des Gewebes - im Vergleich zu näher an der Achse liegenden Bereichen - nicht nur zu einem geringeren Teil in das Gewebe eindringt, sondern dort auch mit einer geringeren wirksamen Strahlungsstromdichte den Fotosensibilisator aktiviert. Dem wird gemäß der Erfindung gezielt Rechnung getragen, indem die Strahlungsstromdichte inhomogen so eingestellt wird, dass z. B. überall, falls dies gewünscht ist, die Vernetzung homogen über das gesamte bestrahlte okulare Gewebe erfolgt.

Die "Strahlungsstromdichte" (*irradiance*) ist hier durchwegs so zu verstehen, dass sie in einer Ebene gemessen wird, die senkrecht steht zur optischen Achse der Mittel zum Einstellen der Verteilung der Strahlungsstromdichte. Die Strahlungstromdichte wird üblicherweise in Watt pro Quadratmeter gemessen.

Die hier vorgenommene Festlegung der Strahlungsstromdichte und Ihrer Verteilung in Bezug auf eine zur optischen Achse A senkrechte Ebene E hat den Vorteil, dass die gewünschte Verteilung der Strahlungsstromdichte sowohl örtlich als auch hinsichtlich der Intensität mit relativ einfachen optischen Mitteln zu bewerkstelligen ist. Auch ist damit bei einem gegebenen System eine Änderung der gewünschten Strahlungsstromdichteverteilung relativ einfach möglich, indem z. B. entweder die optische Komponente ausgetauscht wird oder die optische Komponente so gewählt wird, dass sie änderbar ist. Eine solche einfache Änderung ist insbesondere möglich bei Einsatz eines optischen Elementes in Form einer Flüssigkristallplatte, einer Linse mit Flüssigkeitsbenetzung, oder auch einfach verstellbaren Polarisationsfiltern.

Eine weitere bevorzugte Ausgestaltung der Erfindung sieht vor, dass die genannten Mittel zum Einstellen einer inhomogenen Strahlungsdichteverteilung eine Änderung der Inhomogenität der Verteilung ermöglichen. Mit anderen Worten: Es wird eine Vorrichtung bereitgestellt, die es dem Ophthalmalogen ermöglicht, je nach der Indikation, ohne großen Aufwand mit ein und derselben Vorrichtung nur durch Änderung der optischen Mittel bzw. der Einstellung der gegebenen optischen Mittel, eine Änderung in der radialen und/oder meridionalen Strahlungsdichteverteilung zu bewirken.

Als optisches Element zur Einstellung der Strahlungsstromdichteverteilung der elektromagnetischen Strahlung werden wahlweise eingesetzt: ein absorbierendes optische Element (*neutral density filter*), ein brechendes optisches Element, ein beugendes optisches Element, ein deformierbarer Spiegel, eine Linse mit Elektrobenetzung (*electrowetting lens*), eine Mikrospiegelanordnung (*micro mirror array*), Polarisationsfilter, oder eine Flüssigkristallplatte.

Die Mittel zum Einstellen der elektromagnetischen Strahlungsdichteverteilung können so ausgelegt sein, dass nur ein Teil des okulären Gewebes, insbesondere nur ein Teil der Kornea elektromagnetischer Strahlung ausgesetzt wird. Dies ist nicht nur in Querrichtung (senkrecht zur optischen Achse) zu verstehen, sondern insbesondere auch in Richtung der Strahlung, also im Sinne der Wirktiefe der elektromagnetischen Strahlung im Gewebe. So kann z. B. vorgesehen sein, die elektromagnetische Strahlung mit den genannten Mitteln so einzustellen, dass nur ein äußerer Teil der Kornea bis zu einer bestimmten vorgegebenen Wirktiefe elektromagnetischer Strahlung ausgesetzt wird. Hierzu wird die Intensität der Strahlung entsprechend eingestellt, so dass die Strahlung im Wesentlichen bis zu der gewünschten Wirktiefe vollständig absorbiert ist (durch die Photosensibilisatoren oder andere absorbierende Teile im Gewebe). Die Strahlungsdichte ist also radial und/oder meridional einstellbar (wählbar).

Nachfolgend wird die Erfindung anhand eines Ausführungsbeispieles näher erläutert.

Es zeigt:
- Fig. 1: schematisch die Wechselwirkung zwischen elektromagnetischer Strahlung und einer mit einem Photosensibilisator versehenen Kornea; und
- Fig. 2: eine Vorrichtung zur Erzeugung einer Änderung der biomechanischen Eigenschaften am Beispiel einer Kornea, die einen Photosensibilisator enthält.

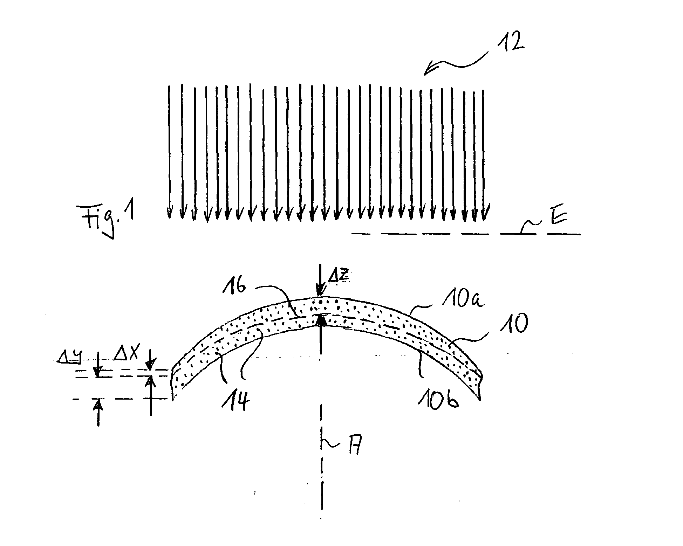

In der schematischen Darstellung gemäß Fig. 1 wird eine Kornea 10 ganzflächig, d. h. über ihre Erstreckung von Limbus zu Limbus, mit elektromagnetischer UV-Strahlung 12 (bei bestimmten bekannten Photosensibilisatoren kommt auch UVA-Strahlung zum Einsatz) bestrahlt. Die Kornea 10 ist in bekannter Weise homogen von einem zuvor eingebrachten Photosensibilisator 14 durchsetzt. Photosensibilisatoren als solche sind im Stand der Technik bekannt, z. B. Riboflavin. Nach Auftropfen auf die Kornea verteilt sich der Photosensibilisator im Wesentlichen gleichmäßig in der gesamten Kornea. Durch die Bestrahlung mit elektromagnetischen Strahlen werden biochemische und biomechanische Prozesse induziert, insbesondere das sogenannte "cross-linking", also eine Vernetzung, welche zu einer Verfestigung der Hornhaut führt, also einer Änderung der biomechanischen Eigenschaften der Hornhaut. Photosensibilisatoren werden als körpereigene Substanz rückstandslos anschließend in relativ kurzen Zeitspannen abgebaut. Die hier eingesetzte elektromagnetische Strahlung im UV-Bereich ist hinsichtlich der Intensität und Wirkung nicht zu verwechseln oder zu vergleichen mit für eine Ablation der Hornhaut eingesetzter elektromagnetischer Strahlung, z. B. bei der LASIK oder Thermokeratoplastik.

Die Intensität der elektromagnetischen Strahlung 12 wird üblicherweise zur Schonung des Gewebes möglichst gering eingestellt. In Fig. 1 ist die Eindringtiefe der elektromagnetischen Strahlung, also ihre sogenannte Wirktiefe, mit der gestrichelten Linie 16 angedeutet. Bei der Linie 16 also ist die in die Kornea 10 eingestrahlte elektromagnetische Strahlung 12 im Wesentlichen absorbiert und hat anschließend, d. h. in tieferen Bereichen der Kornea nahe deren Rückseite 10b keine Wirkung mehr. Die Vorderseite der Kornea ist in den Figuren mit 10a bezeichnet.

Wie Fig. 1 zeigt, ist die durch die Linie 16 angedeutete Wirktiefe der elektromagnetischen Strahlung hinsichtlich der Aktivierung des Photosensibilisators abhängig vom Ort der Einstrahlung in die Kornea 10. Die Wirktiefe nimmt radial nach außen, ausgehend von einer Augenachse A ab. Beim in Fig. 1 dargestellten Ausführungsbeispiel ist die Intensität der eingestrahlten Strahlung 12, genauer: Die Strahlungsstromdichte (auch Bestrahlungsstärke, *irradiance,* genannt) in einer Ebene E senkrecht zur Augenachse A homogen, d. h. in der Ebene E gleichbleibend. Die vorliegende Erfindung beruht auf der Erkenntnis, dass trotz der homogenen Verteilung der Strahlungsstromdichte in der Ebene E die Wirktiefe der Strahlung im den Photosensibilisator enthaltenen Korneagewebe 10 radial nach außen abnimmt, d. h. mit zunehmenden Abstand von der Augenachse A abnimmt. Während die Wirktiefe Δ Z der Strahlung im Bereich der Augenachse A beim dargestellten Ausführungsbeispiel aufgrund der Intensität der elektromagnetischen Strahlung 12 etwa bis zur Mitte der Korneastärke reicht, ist die Wirktiefe Δx am peripheren Rand der Kornea 10 deutlich geringer und entsprechend der Bereich Δy, in dem die elektromagnetische Strahlung keine wesentliche Wirkung mit dem Photosensibilisator erzielt, deutlich größer als die halbe Stärke der Kornea.

Die Erfindung beruht auf der Erkenntnis dieser Zusammenhänge, die sich wie folgt erklären:
Die Wirkung der Strahlung im Korneagewebe hängt von der lokalen Strahlungsstromdichte (typisches Symbol "E"; definiert als die Leistung der eingehenden elektromagnetischen Energie pro Flächeneinheit) ab. Bei der in Fig. 1 dargestellten Situation hängt die Strahlungsstromdichte im Wesentlichen von zwei Effekten ab, zum einen vom Winkel zwischen der Strahlung und der Oberfläche 10a der Kornea 10 und zum anderen von der Reflexion von Strahlung an der Oberfläche 10a. Aufgrund der gegebenen relativen Winkel zwischen der Einstrahlungsrichtung der Strahlung und der Oberfläche 10a der Kornea wird von der zentralen Augenachse A ausgehend radial nach außen zur Peripherie der Kornea immer mehr Strahlung reflektiert (weil von der Mitte ausgehend der Winkel von anfänglich etwa 90° immer spitzer wird). Zum anderen wird auch die Strahlungsstromdichte radial nach außen immer kleiner aufgrund des Winkels zwischen dem Gewebe und der einfallenden Strahlung. Zusätzlich zu diesem Aspekt wird die Wirktiefe nach peripher noch stärker reduziert, weil der Zusammenhang zwischen Strahlungsintensität und Wirktiefe/Vernetzungsgrad nichtlinear ist. Diese Effekte addieren sich und führen zu der in Fig. 1 dargestellten Verringerung der Wirktiefe 16 der Strahlung mit zunehmendem radialen Abstand von der Augenachse A.

Der Begriff "Augenachse" ist in der Ophthalmologie unterschiedlich definiert. Im Zusammenhang mit der vorliegenden Erfindung kommt es nicht ganz genau darauf an, welche Definition der "Achse" herangezogen wird. Man kann sich unter der Achse hier z. B. die optische Achse des optischen Systems "Auge" vorstellen, also eine Gerade, welche die Krümmungsmittelpunkte der brechenden Flächen in einem zentrierten System verbindet. Bei in den Figuren dargestellten Anordnungen fällt die optische Achse des Auges auch mit der optischen Achse des die Strahlung aufbringenden optischen Systems zusammen. Im Zusammenhang mit dieser Beschreibung betrifft der Begriff "radial" die Entfernung von der Achse A in senkrechter Richtung hierzu und der Begriff "axial" eine Richtung parallel zur Achse A. Die Ebene E verläuft also radial.

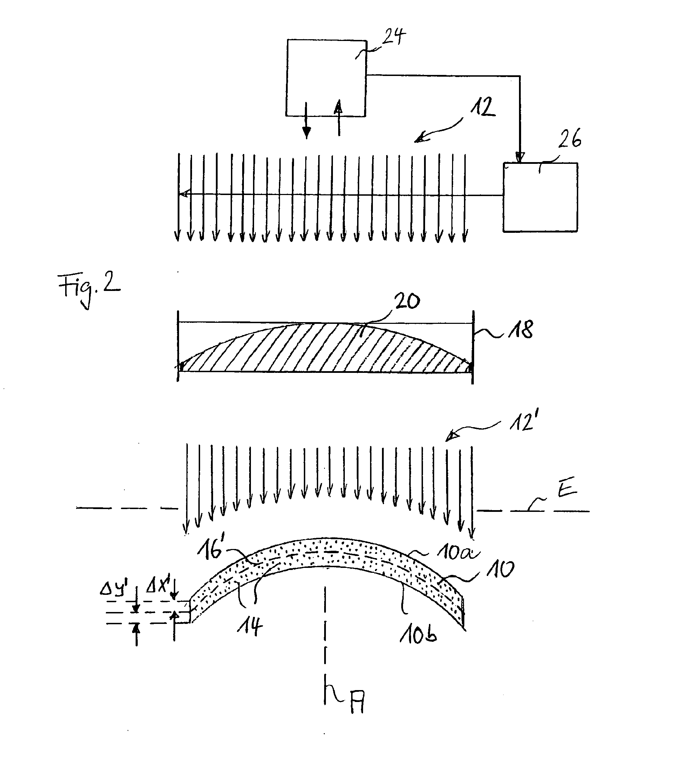

Fig. 2 zeigt eine weitere Vorrichtung zur Erzeugung einer Änderung der biomechanischen Eigenschaften okulären Gewebes, hier in Form einer Kornea 10. Die elektromagnetische Strahlung 12 passiert vor ihrem Auftreffen auf die Kornea 10 Mittel 18, 20, mit denen die Wirktiefe der elektromagnetischen Strahlung im okulären Gewebe einstellbar ist. In Fig. 2 ist die Strahlungsstromdichte der elektromagnetischen Strahlung 12 durch die Länge der Pfeile dargestellt. Die elektromagnetische Strahlung 12 wird durch eine bekannte Strahlungsquelle (nicht dargestellt) erzeugt. Die Mittel 18, 20 zum Einstellen der Strahlungsstromdichteverteilung weisen ein Gehäuse 18 auf, in dem beim dargestellten Ausführungsbeispiel ein Strahlungsabsorber 20 (*neutral density filter*) wahlweise angeordnet ist. Die Absorptionsplatte ist in Fig. 2 nur schematisch dargestellt. Sie absorbiert ausschließlich die Strahlung, ohne Abbildungseigenschaften zu bewirken, also insbesondere ohne eine Richtungsänderung von der einfallenden zur ausfallenden Strahlung zu erzeugen. Die Schraffur im Element 20 gemäß Fig. 2 entspricht der absorbierenden Stärke des Elementes, die radial nach außen abnimmt.

Nach passieren des Elementes 20 hat die elektromagnetische Strahlung die Strahlungsstromdichteverteilung 12', bei der die Länge der Pfeile jeweils die örtliche Strahlungsstromdichte schematisch darstellt. Mit dieser Strahlungsstromdichteverteilung, bei der die Strahlungsstromdichte radial mit ihrem Abstand von der Achse A zunimmt, wird eine Wirktiefe bezüglich der Aktivierung des Photosensibilisators 14 in der Kornea 10 gemäß der Linie 16' erreicht. Beim dargestellten Ausführungsbeispiel wird angestrebt, eine Wirktiefe entsprechend der Linie 16' über die gesamte radiale Erstreckung der Kornea 10 im Wesentlichen in der Mitte der axialen Erstreckung der Kornea zu erreichen. Entsprechend ist die in axialer Richtung gemessene Wirktiefe Δ x' etwa gleich der Stärke Δy' des Bereichs der Kornea, in dem keine wesentliche Änderung der biomechanischen Eigenschaften der Kornea erfolgt.

Die Wirktiefe der elektromagnetischen Strahlung und der damit verbundenen Vernetzung des Gewebes lässt sich mit der optischen Kohärenztomographie bestimmen. Es versteht sich, dass die Darstellungen und Beschreibungen der Figuren 1 und 2 um die Achse A rotationssymmetrisch sind, d. h. die dargestellten Schnitte gelten schematisch für jede Richtung eines Schnittes senkrecht zur Achse A, der auch die Achse A enthält. Die erfindungsgemäße Vorrichtung ist also so eingerichtet, dass die (für die jeweilige medizinische Indikation benötigte) Inhomogenität der elektromagnetischen Strahlung meridional und radial einstellbar ist.

Als Mittel 20 zum Einstellen der Strahlungsstromdichteverteilung der elektromagnetischen Strahlung 12' kommen außer dem vorstehend beispielhaft beschriebenen Strahlungsabsorber in Betracht: ein brechendes optisches Element, ein beugendes optisches Element, ein deformierbarer Spiegel, eine Linse mit Elektrobenetzung (*electrowetting lens*), eine Mikrospiegelanordnung (*micro mirror array*), Polarisationsfilter, oder eine Flüssigkristallplatte oder eine Kombination eines oder mehrerer dieser Elemente.

Mit der Erfindung ist es möglich, eine Änderung des Augengewebes bezüglich seiner Gestalt und/oder zumindest einer mechanischen Eigenschaft zu bewirken, wobei im Gewebe eine Lichtverteilung mit mindestens einem optischen Element 20 erzeugt wird, welche zu einer vorab definierten Verteilung der Quervernetzungen in der Tiefe der Gewebestrukturen führt. Die örtliche (geometrische) Verteilung der Vernetzungen wird dabei durch Einsatz des Elementes 20 bzw. mehrerer Elemente an dieser Stelle wahlweise radial, axial und/oder meridional in Bezug auf die Vorderfläche 10a oder die Rückfläche 10b der Kornea 10 gesteuert. Die Lichtverteilung (Strahlungsstromdichteverteilung) wird insbesondere so gesteuert, dass Projektionseffekte und Reflexionsverluste der Strahlung berücksichtigt sind. Die Erfindung ermöglicht also durch Variation der Strahlungsintensität und/oder Strahlungsdosis eine gezielte Neuformung der Kornea im Wechselspiel zwischen den biomechanischen Eigenschaften der Kornea und dem Augeninnendruck.

Fig. 2 zeigt auch eine Weiterentwicklung des Erfindungsgedankens unter Einschluss von Mitteln (24) zur Bestimmung der aktuellen während des Einstrahlens elektromagnetischer Strahlung erzeugten Wirktiefe im Gewebe, z. B. der Kornea (10). Zum Beispiel ist als Mittel zum Bestimmen der Wirktiefe ein als solches bekanntes Gerät der optischen Kohärenztomographie (OCT) geeignet. Eine solche OCT-Einrichtung ist in Fig. 2 schematisch mit dem Bezugszeichen 24 bezeichnet. Mittel (26) sind vorgesehen, um die elektromagnetische Strahlung in Raum und Zeit zu steuern. Auch solche Mittel zum Steuern elektromagnetischer Strahlung in Raum und Zeit sind in der ophthalmalogischen Technik weithin bekannt.

Mit den Mitteln 24 für die optische Kohärenztomographie wird während der Einstrahlung elektromagnetischer Strahlung 12 die jeweils erreichte Wirktiefe 16' "online" bestimmt und entsprechend der bestimmten Wirktiefe und einem Vergleich mit einer Solltiefe kann dann die Einrichtung 26 zum Steuern der elektromagnetischen Strahlung 12 angesteuert werden, um eine gewünschte End-Wirktiefe zu erreichen.

Die für einen Patienten zu erzielende End-Wirktiefe kann z. B. empirisch gewonnen werden, in Abhängigkeit von der Diagnose und empirischen oder abgeschätzten Daten für die anzustrebende Therapie mittels Änderung der biomechanischen Eigenschaften des Gewebes. Zum Beispiel kann die Wirktiefe empirisch mittels einer Vielzahl von Schweineaugen, die mit dem gewählten Photosensibilisator versehen sind und bei denen die Strahlungsstromdichte im Rahmen einer Versuchsreihe variiert wird und wobei dann jeweils die Wirktiefe mittels Kohärenztomographie gemessen wird, bestimmt werden. Der Begriff "Wirktiefe" bezeichnet die Strecke, in der, ausgehend von der Vorderfläche der Kornea, die biomechanischen Eigenschaften des Gewebes durch das Zusammenwirken des Photosensibilisators mit der Strahlung im Vergleich zum Ausgangszustand geändert sind. Damit ergibt sich ein sogenanntes Profil für die Wirktiefe, d. h. eine Funktion der Wirktiefe in Abhängigkeit vom Ort im Gewebe. Beim schematischen Ausführungsbeispiel gemäß Fig. 2 ist die End-Wirktiefe 16' gleichmäßig etwa mittig in der Kornea, wie oben beschrieben. Bei bestimmten Indikationen kann es aber erwünscht sein, eine ungleichmäßige End-Wirktiefe derart zu erreichen, dass sie nicht den in Fig. 2 schematisch gezeigten gleichförmigen Verlauf in der Mitte der Kornea hat, sondern als Funktion des Abstandes von der zentralen Achse A variiert, z. B. so, dass, je nach Indikation, in der Mitte oder peripher die End-Wirktiefe näher oder weiter an der Vorderseite 10a bzw. der Hinterseite 10b der Kornea verläuft.

Die End-Wirktiefe kann, je nach Diagnose und Indikation, z. B. empirisch unter Verwendung der sogenannten Topographiedaten (Vermessung der Oberfläche 10a der Kornea), der Hornhautstärke, und mit Hilfe von Wellenfrontmessungen, ermittelt werden.

## Patentansprüche

1. Vorrichtung zur Erzeugung und Änderung von biomechanischen Eigenschaften okulären Gewebes (10), das Photosensibilisator (14) enthält, mit Mitteln (18, 20) zum Einstrahlen von elektromagnetischer Strahlung (12') in das Gewebe (10), die mit dem Photosensibilisator (14) eine Vernetzung bewirkt und mit Mitteln (18, 20), die eingerichtet sind zum Einstellen einer inhomogenen Verteilung der Strahlungsstromdichte der elektromagnetischen Strahlung (12'), **dadurch gekennzeichnet, dass** die Mittel (18, 20) zum Einstellen einer inhomogenen Strahlungsstromdichteverteilung eingerichtet sind, in weiter von ihrer zentralen Achse entfernten Bereichen die elektromagnetische Strahlung mit einer höheren Strahlungsstromdichte in das okuläre Gewebe einzustrahlen als in näher an der Achse (A) liegenden Bereichen, wobei als Mittel für die Einstellung der Strahlungsstromdichteverteilung der elektromagnetischen Strahlung eines oder mehrere der folgenden optischen Einrichtungen vorgesehen ist bzw. sind:
ein absorbierendes optische Element (*neutral density filter*), ein brechendes optisches Element, ein beugendes optisches Element, ein deformierbarer Spiegel, eine Linse mit Elektrobenetzung (*electrowetting lens*)*,* eine Mikrospiegelanordnung (*micro mirror array*), eine Flüssigkristallplatte oder Polarisationsfilter.

2. Vorrichtung nach Anspruch 1, **dadurch gekennzeichnet, dass** die Mittel (18, 20) eingerichtet sind die Kornea (10) ganzflächig zu bestrahlen.

3. Vorrichtung nach einem der vorhergehenden Ansprüche, **dadurch gekennzeichnet, dass** die inhomogene Verteilung der Strahlungsstromdichte der elektromagnetischen Strahlung (12') in einer Ebene (E) senkrecht zur optischen Achse (A) der Mittel (18, 20) zum Einstellen einer inhomogenen Verteilung der Strahlungsstromdichte vorliegt.

4. Vorrichtung nach einem der vorhergehenden Ansprüche, **dadurch gekennzeichnet, dass** die Mittel (18, 20) zum Einstellen einer inhomogenen Verteilung der Strahlungsstromdichte für eine Änderung der Inhomogenität der Verteilung der Strahlungsstromdichte der elektromagnetischen Strahlung eingerichtet sind.

## Claims

1. Apparatus for generation and alteration of biomechanical properties of ocular tissue (10) that contains photosensitizer (14), comprising means (18, 20) for radiating into the tissue (10) electromagnetic radiation (12') which with the photosensitizer (14) brings about a cross-linking, and comprising means (18, 20) that are configured for setting an inhomogeneous distribution of the irradiance of the electromagnetic radiation (12'), **characterized in that** the means (18, 20) for setting an inhomogeneous distribution of the irradiance are configured to radiate the electromagnetic radiation into the ocular tissue with a higher irradiance in regions further away from their central axis than in regions situated closer to the axis (A), wherein one or more of the following optical elements is provided as means for setting the distribution of the irradiance of the electromagnetic radiation:
an absorbing optical element (neutral density filter), a refracting optical element, a diffracting optical element, a deformable mirror, an electrowetting lens, a micromirror array, a liquid-crystal plate or polarizing filters.

2. Apparatus of claim 1, **characterized in that** the means (18, 20) are configured for irradiating the cornea (10) over its entire surface.

3. Apparatus of any one of the previous claims, **characterized in that** the inhomogeneous distribution of the irradiance of the electromagnetic radiation (12') is in a plane (E) that is perpendicular to the optical axis (A) of the means (18, 20) for setting the inhomogeneous distribution of the irradiance.

4. Apparatus of any one of the previous claims, **characterized in that** the means (18, 20) for setting the inhomogeneous distribution of the irradiance are configured for alteration of the inhomogeneity of the distribution of the irradiance of the electromagnetic radiation.

## Revendications

1. Dispositif de production et de modification des propriétés biomécaniques d'un tissu oculaire (10) qui contient un photosensibilisateur (14), comportant des moyens (18, 20) pour émettre dans le tissu (10) un rayonnement électromagnétique (12') qui provoque une réticulation avec le photosensibilisateur (14), et comportant des moyens (18, 20) qui sont adaptés pour régler une répartition non homogène de la densité de puissance du rayonnement électromagnétique (12'), **caractérisé en ce que** les moyens (18, 20) de réglage d'une répartition non homogène de la densité de puissance sont adaptés pour émettre le rayonnement électromagnétique dans le tissu oculaire avec une densité de puissance plus élevée dans des zones plus éloignées de leur axe central que dans des zones plus proche de l'axe (A), le ou les moyen(s) de réglage de la répartition de densité de puissance du rayonnement électromagnétique étant formé(s) par un ou plusieurs des dispositifs optiques suivants :
un élément optique absorbant (*neutral density fi*/*ter*), un élément optique réfractant, un élément optique diffractant, un miroir déformable, une lentille à électro-mouillage (*electrowetting lens*), un réseau de micro-miroirs (*micro mirror array*), un panneau à cristaux liquides ou un filtre polarisant.

2. Dispositif selon la revendication 1, **caractérisé en ce que** les moyens (18, 20) sont adaptés pour irradier la cornée (10) sur toute la surface.

3. Dispositif selon l'une des revendications précédentes, **caractérisé en ce que** la répartition non homogène de la densité de puissance du rayonnement électromagnétique (12') est présente dans un plan (E) perpendiculaire à l'axe optique (A) desdits moyens (18, 20) de réglage d'une répartition non homogène de la densité de puissance.

4. Dispositif selon l'une des revendications précédentes, **caractérisé en ce que** les moyens (18, 20) de réglage d'une répartition non homogène de la densité de puissance sont adaptés pour permettre une modification de la non-homogénéité de la répartition de la densité de puissance du rayonnement électromagnétique.
